# EUROPEAN PATENT SPECIFICATION

(11) **EP 0 987 937 B1**
(45) Date of publication and mention of the grant of the patent: **03.03.2004**
(21) Application number: 98928038.3
(22) Date of filing: 12.06.1998
(51) Int. Cl.: A01H 4/00

(54) **MEDIA AND METHODS FOR CULTURING PLANT EMBRYOS**
VERFAHREN ZUR KULTIVIERUNG PFLANZLICHER EMBRYONEN
MILIEUX ET METHODES PERMETTANT DE CULTIVER DES EMBRYONS VEGETAUX

(30) Priority: 13.06.1997 US 50014 P
(43) Date of publication of application: 29.03.2000
(73) Proprietor: University of Saskatchewan, Saskatoon, Saskatchewan S7N 5C8 (CA)
(72) Inventor: ILIC-GRUBOR, Katica, Saskatoon, Saskatchewan S7K 2J7 (CA); ATTREE, Stephen, M., Victoria, British Columbia V8S 5B1 (CA); FOWKE, Lawrence, C., Saskatoon, Saskatchewan S7J 0J3 (CA)
(74) Representative: Bannerman, David Gardner
(86) International application number: PCT/CA1998/000582
(87) International publication number: WO 1998/057536

(56) References cited:
- WO-A-93/11660
- US-A- 5 350 688
- PATENT ABSTRACTS OF JAPAN vol. 014, no. 299 (C-0733), 27 June 1990 & JP 02 097341 A (MITSUI PETROCHEM IND LTD), 9 April 1990
- PATENT ABSTRACTS OF JAPAN vol. 096, no. 009, 30 September 1996 & JP 08 131000 A (HOUSE FOODS CORP), 28 May 1996
- DATABASE CAB CAB INTERNATIONAL, WALLINGFORD, OXON, GB AN- 93:63622, 1993 MCCLELLAN, D.: "Glucosinolate metabolism in zygotic and microspore-derived embryos of Brassica Napus" XP002093872
- RANU DE ET AL.: "Effect of water stress on protein, amino acids and proline contents in germinating mungbean seeds" INDIAN JOURNAL OF PLANT PHYSIOLOGY, vol. 37, no. 2, 1994, pages 116-118, XP002083930

## Description

### TECHNICAL FIELD

The present invention relates generally to the field of plant embryogenesis, and more particularly to methods for culturing plant embryos that closely resemble embryos which have undergone natural development.

### BACKGROUND OF THE INVENTION

Artificially generated embryos are commonly used to produce a variety of plants for genetic manipulations and developmental studies. In *Brassica napus,* for example, haploid embryo formation from isolated microspores was first reported by Lichter, *Z. Pflanzenphysiol 105*:427-434, 1982. Since then, remarkable progress has been made in developing systems for producing microspore-derived (MD) embryos in this rapeseed species (Chuong and Beversdorf, *Plant Sci. 39*:219-226, 1985; Pechan and Keller, *Physiol. Plant 74*:377-384, 1988; Telmer et al., *Physiol. Plant 84*:417-424, 1992). Such systems have been used for selection in oilseed rape breeding programs (Keller et al., *in*: Green et al. (eds), *Plant tissue and cell culture, Proc. 6*^{*th*} *Int. Plant Tissue Culture Cong.,* pp. 223-241, 1987 (Alan R Liss Inc., New York); Polsoni et al., *Can. J. Bot. 66*:1681-1685, 1988; Swanson et al., *Plant Cell Rep. 7*:83-87, 1988) and for various genetic manipulations (reviewed by Huang, *In Vitro Cell. Dev. Biol. 28P*:53-58, 1992).

Commonly used protocols for producing embryos of *Brassica napus* and other species involves culturing in media containing relatively high levels of sucrose (usually 3-17%) as both osmoticum and carbon source (Keller et al., *Can. J. Genet. Cytol. 17*:655-666, 1975; Lichter, *Z Pflanzenphysiol 103:* 229-237, 1981; Lichter, *Z*. *Pflanzenphysiol 105*:427-434, 1982; Dunwell and Thurling, *J. Exp. Bot. 36:* 1478-1491, 1985; Roulund et al., *Euphytica 52*: 125-129, 1991; Baillie et al., *Plant Cell Rep. 11*:234-237, 1992; Ferrie et al., *in:* Thorpe (ed), *In vitro Embryogenesis in Plants,* pp. 309-344, 1995 (Kluwer Academic Publishers, Dordrecht Netherlands)). As a metabolite, sucrose can be absorbed into cell symplasts, readily utilized and/or stored in vacuoles or converted into starch and stored in plastids. Thus, in the case of *Brassica napus,* the high levels of sucrose used appear to result in substantial sugar uptake by the embryos, leading to abundant accumulation of starch (Zaki and Dickenson, *Sexual Plant Reproduction 4*:48 55, 1991; Yeung et al., *Int. J. Plant Sci. 157*:27-39, 1996) and lipids (Taylor et al., *Planta 181*:106-112, 1990), as well as abnormal enlargement and external morphology (*e*.*g*., cotyledon morphology (Hause et al., *Botanica Acta 107:407-415,* 1994)). Such effects can be observed within 3-4 weeks of culture, at which time the immature embryos differ significantly from zygotic embryos at a comparable stage. In particular, the immature embryos are considerably larger, have a different morphology and contain large amounts of starch. When germinated, conversion frequencies are poor and many embryos form abnormal plants. As a result, enormous numbers of embryos must be plated in order to generate useful numbers of plants.

US 5,350,688 discloses culturing embryos in the presence of a carbon source and a water stressing agent.

Accordingly, there is a need in the art for techniques that improve the efficiency of normal plant embryo production. The present invention fulfills this need, and further provides other related advantages.

### SUMMARY OF THE INVENTION

Briefly stated, the present invention provides media and methods according to claims 1 and 23 for the production of plant embryos. Embryos produced by these methods more closely resemble their counterpart normal zygotic embryos which have undergone natural development than do embryos produced in accordance with the methods of the prior art. Similarly, the media formulation provided in the present invention may be used in accordance with the methods provided Within one variant of the present invention, methods are provided for culturing plant embryos comprising developing plant embryos in media comprising a metabolizable carbon source in a concentration of that does not exceed the amount necessary to promote embryo development. In general, the concentration of the metabolizable carbon source to be used should be at least sufficient to meet the minimum nutritional requirements of the embryos, but no more than about that which is necessary to promote the normal development processes of the embryos. To determine the appropriate concentration of metabolizable carbon source, an empirical approach should be employed, taking into account such variables as the type of metabolizable carbon source, the species of the embryos, and so forth. For example, if an easily metabolized carbon source (such as, for example, sucrose) is used, the concentration should preferably be less than about two percent (and preferably less than about 0.75% to 1%), under conditions and for a time sufficient to promote embryo development. On the other hand, if a poorly metabolized carbon source (such as, for example, sugar alcohols including sorbitol and mannitol) is used, it may be necessary to raise the concentration. In this latter example, it is to be understood that the concentration of the poorly metabolized carbon source should be limited to minimize the toxic effects resulting from embryo uptake.

Methods are provided for culturing plant embryos, comprising developing plant embryos in media comprising a metabolizable carbon source in a concentration of less than about two percent and water stressing the embryos in a water potential greater than 300 mmol/kg, under conditions and for a time sufficient to promote embryo development. Although it is contemplated in this variant that the intensity of the water stressing should be sufficient to create a water potential at least equal to that created in the prior art by higher concentrations of metabolizable carbon sources, the water potential may be even greater than about 750 mmol/kg, depending upon the species being cultured. The water stressing may be effected by any means of simulating drought stress (other than, of course, additional amounts of metabolizable carbon sources) such as one or more osmotica, environments of relative humidity with differing water potentials than the embryos, and so forth. It is to be understood that more than one means of water stressing may be used at any given time, and also that different means may be used at different times during development. Furthermore, it is to be understood that the period during which water stressing is to occur should be selected with such factors as the source of the embryos in mind. For example, microspore-derived embryos may be water stressed from the beginning of the culture period, whereas, for somatic embryos, it is not necessary to commence water stressing until one wishes to initiate the development process.

In the second variant of the method, preferred osmotica include non-permeating osmotica such as polyalkylene glycols (*e*.*g*., PEG) having a molecular weight of greater than about 950, or dextrans of sufficiently high molecular weight (*e*.*g*., Dextran 6000). The preferred concentrations (w/v) of non-permeating osmotica range between about 7% and 30%. If, however, water stress is exerted by means of a gelling agent, such as, for example, Phytagel, the concentration of the gelling agent must be determined empirically, based on the type of gelling agent and the brand.

A wide variety of metabolizable carbon sources may be utilized within the context of the present invention, including, for example, fructose, glucose, maltose, myoinositol, and sucrose. The concentration of the metabolizable carbon source in the medium may preferably range from about 0.01% to about 1.5%, but may be even less than about 0.01%. However, if a poorly metabolized carbon source such as sugar alcohols (for example, sorbitol and mannitol) are used, an empirical approach should be taken to ensure that the concentrations used are limited to minimize the toxic effects when taken up by the embryos. As utilized within the context of the present invention, the concentration of the metabolizable carbon source may be determined either based upon a one-time addition of the carbon source, or over the period of several days to a month or more (in this regard the metabolizable carbon source may be continually exchanged utilizing, for example, a bioreactor).

A further variant of the present invention is the formulation of media that may be used to develop the embryos in accordance with the foregoing methods. The media comprise less than about 2% of an easily metabolized carbon source (such as glucose, sucrose, fructose, and myo-inositol) in combination with a sufficient concentration of non-permeating osmotica to effect a water potential of at least about 300 mmol/kg. The osmotica should preferably be present in a concentration of at least about 7%, and even as high as about 30%. As discussed in the context of the methods of the present invention, the choice of metabolizable carbon source, osmotica, and their respective concentrations should be made in light of such factors as the source of the embryos, the species of the embryos, and type of the carbon source and osmoticum.

The above-described media and methods may be utilized for culturing a wide variety of embryos, including both monocot or dicot (Plant Tissue Culture Concepts and Laboratory Exercises, Ed. R.N. Trigiano and D.J. Gray, Publ. CRC Press, Boca Raton, 1996; Plant Cell, Tissue and Organ Culture: Fundamental Methods, ed. O.L. Gamborg and G.C. Phillips, Sprnger, NY, 1995) angiosperm species. Further, the embryos may be microspore-derived, somatic, or zygotic embryos. Representative examples of suitable plants include the cereals, grasses, *Hevea basiliensis* (rubber trees), aspens, poplars, oaks, walnuts, cotton, alders, various species of eucalyptus, and various species of gymnosperms (*e.g.*, conifers such as pines, spruces, douglas fir, larch, hemlock, yew and cedar).

Although the following examples illustrate the of the present invention in the absence of growth regulators, it is to be understood that growth regulators may be used where appropriate to enhance embryo quality and conversion frequency. For instance, abscisic acid (or other stress hormones) may be used to further the maturation process and enhance the desiccation tolerance of the mature embryos, as taught in the prior art (see, for example, Attree et al. in U.S. patent no. 5,464,769 WO 93/11660 issued on 7 November 1995, and Redenbaugh et al. in U.S. Patent 4,777,762, issued on 18 October 1988). Similarly, other growth regulators may be used, the selection of the particular growth regulator(s) depending on the species and source of the embryos.

As noted earlier, methods in the prior art result in the abnormal enlargement and morphology of embryos cultured with higher concentrations of metabolizable carbon. As discussed in the examples below, it appears that normal morphology is a better indicator than the size of the embryos of plantlet viability and vigor. That is, embryos that are morphologically similar to normal zygotic embryos at the corresponding stage of development tend are more likely to convert to plantlets having good vigor and viability. Thus, relative to this prior art, the methods and media of the present invention result in mature embryos having improved morphology leading to normal (that is, comparable to zygotic) accumulation of storage reserves and improved desiccation tolerance. Also, in the context of microspore-derived embryos, the present invention results in improved induction frequencies. Moreover, the mature embryos convert in increased frequencies to viable plantlets, which plantlets have improved vigor over those in the prior art.

These and other aspects of the present invention will become apparent upon reference to the following detailed description. All references disclosed herein are hereby incorporated by reference in their entirety as if each was incorporated individually.

### BRIEF DESCRIPTION OF THE DRAWINGS

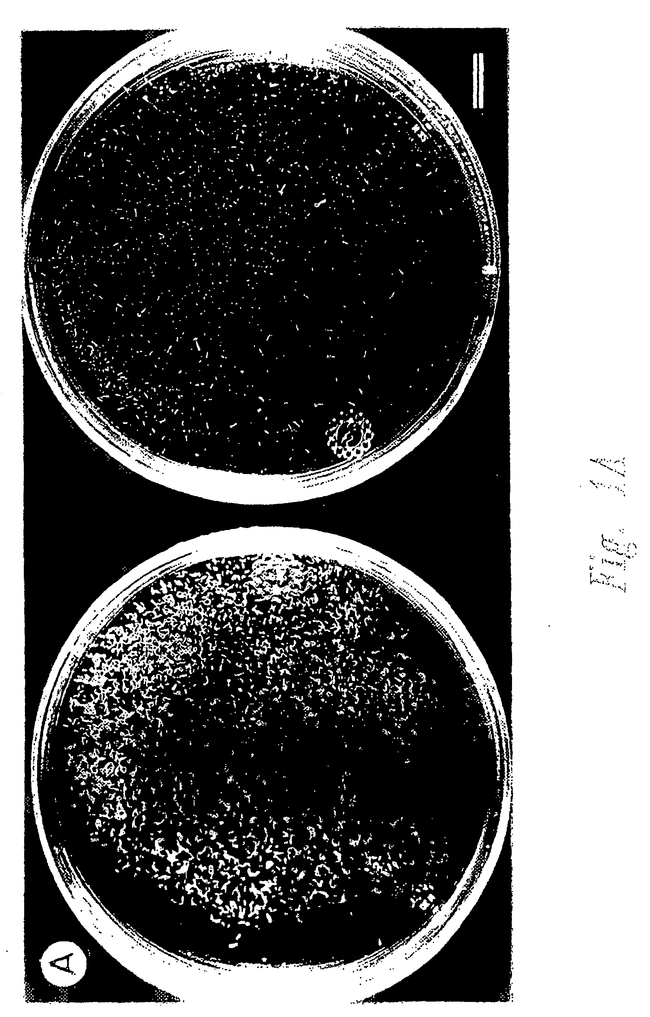

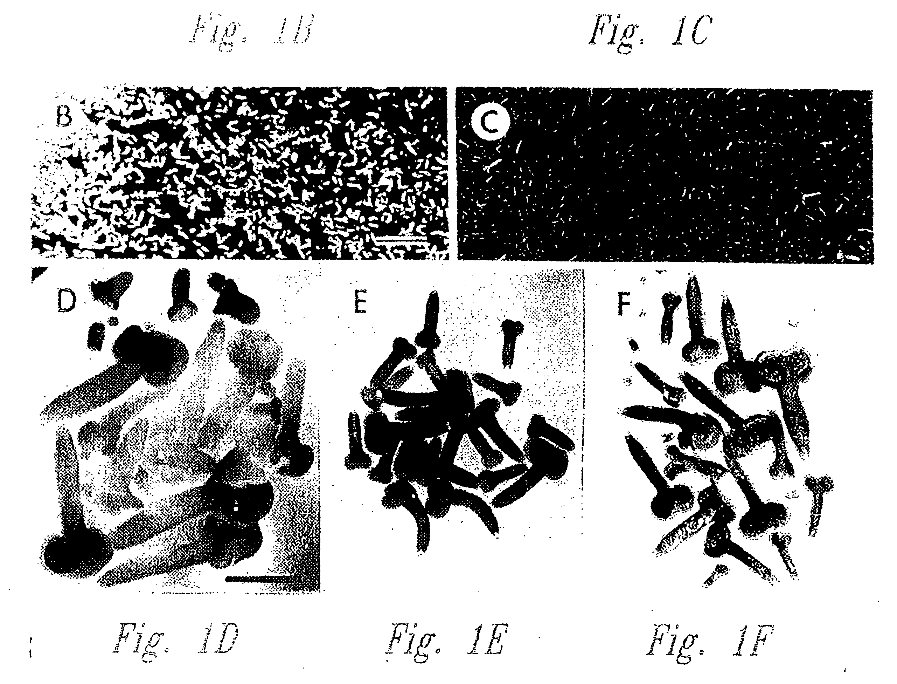

Figures 1A-1F are photographs of microspore-derived embryos. Figure 1A shows sixteen-day old microspore-derived embryos of *Brassica napus* on different types of osmoticum: (a) sucrose (left) and (b) polyethylene glycol 4000 (right). Plates were kept under the light for two days. Bar = 10 mm. Figure 1B is an enlargement of sucrose embryos (from Fig 1A); Figure 1C is an enlargement of PEG embryos (from Fig 1A); bar = 5 mm. Figure 1D shows three-week-old sucrose embryos; figure 1E shows zygotic immature embryos developed *in ovulo*, dissected at 14 and 15 DPA; and Figure 1F shows three week-old PEG embryos (bar = 1 mm).

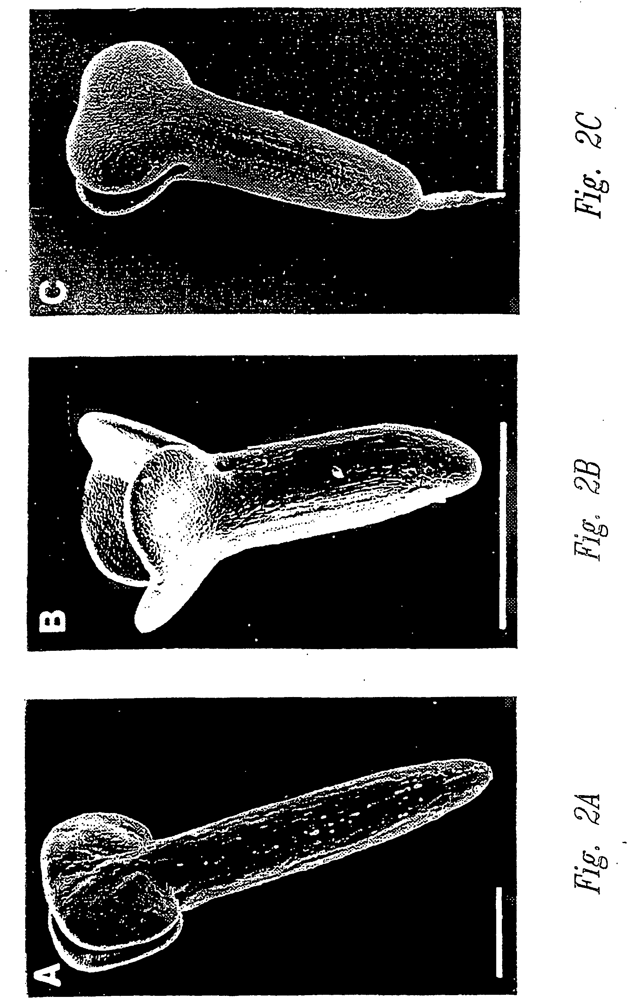

Figures 2A-2C are scanning electron micrographs of early cotylenonary stage embryos of *Brassica napus.* The embryos in Figure 2A were developed on sucrose medium for two weeks, those in Figure 2B were developed on PEG medium for two weeks and those in Figure 2C were dissected immature zygotic embryos developed *in ovulo* (dissected at 15 DPA).

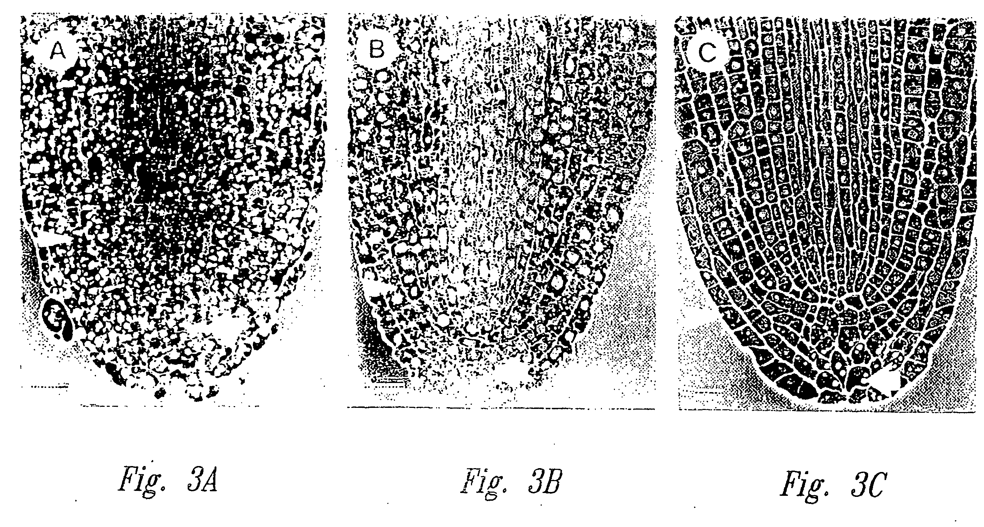

Figures 3A-3C are light micrographs of root apices at the early cotyledon stage. Figure 3A shows a two-week old embryo developed on sucrose medium, Figure 3B shows a zygotic embryo developed *in ovulo* (15 DPA) and Figure 3C shows a two week old embryo developed on PEG medium. Lipid bodies are stained black with Sudan B (arrowheads), while starch grains remain unstained. Scale bars = 20 µm.

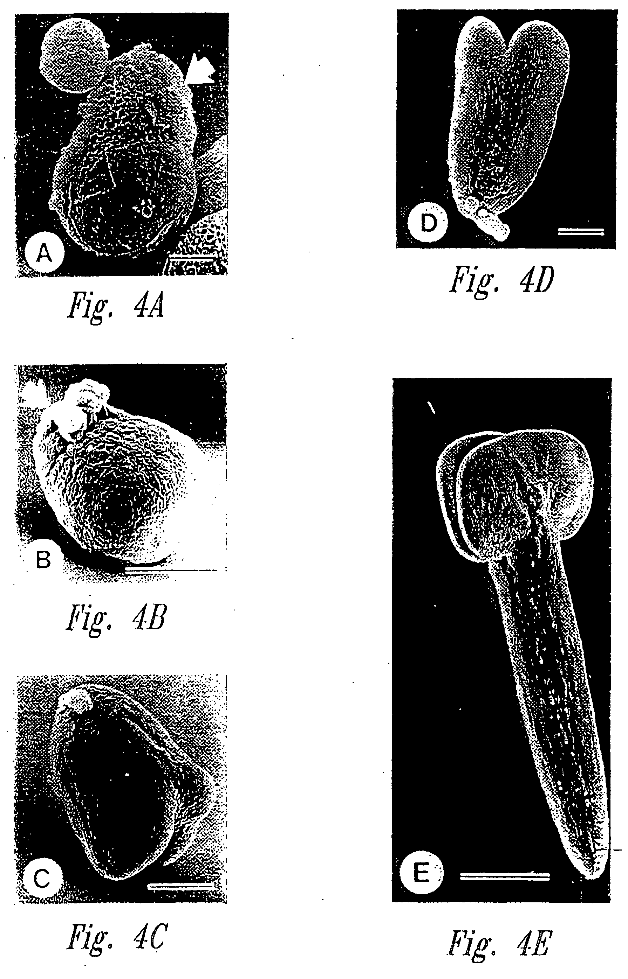

Figures 4A-4J are scanning electron micrographs showing the developmental stages of MD embryos on sucrose medium (A-E) and PEG medium (F-J). Figure 4A shows globular cluster of cells rapidly enlarging upon the break down of pollen wall. Note the 'pocket-like' region with tightly adhered remnants of pollen wall (arrow); also, note size of a dead microspore (arrowhead). Bar =10 µm. Figure 4B shows an oblong proembryo with flattened terminal end and suspensor-like appendices on the basal end (arrows). Figure 4C shows a heart-shaped embryo, and 4D shows early torpedo embryos. Bar =100 µm. Figure 4E shows a two-week old sucrose embryo at early cotyledonary stage. Note abrasion of epidermis near the radicle. Bar = 500 µm.

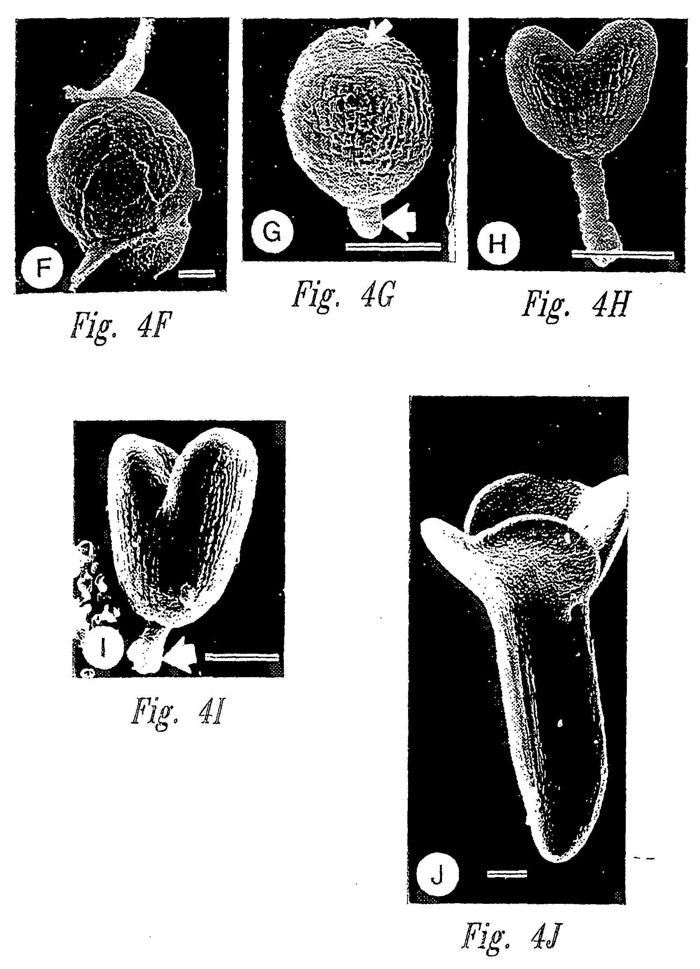

Figure 4F shows a globular proembryo shortly after breakdown of pollen wall. Note remnants of pollen wall on one end of globular proembryo and on opposite side. Bar = 10 µm. Figure 4G shows an oval-shaped embryo with concave terminal end and suspensor-like protrusion at the basal end (arrows). Figure 4H shows a heart-shaped embryo with the long filamentous suspensor, and Figure 41 shows an early torpedo embryo with multicellular tip of suspensor (arrow) and dead microspores (arrowhead). Figure 4J shows an early cotyledon stage embryo after two weeks in culture. Bar = 1000 µm.

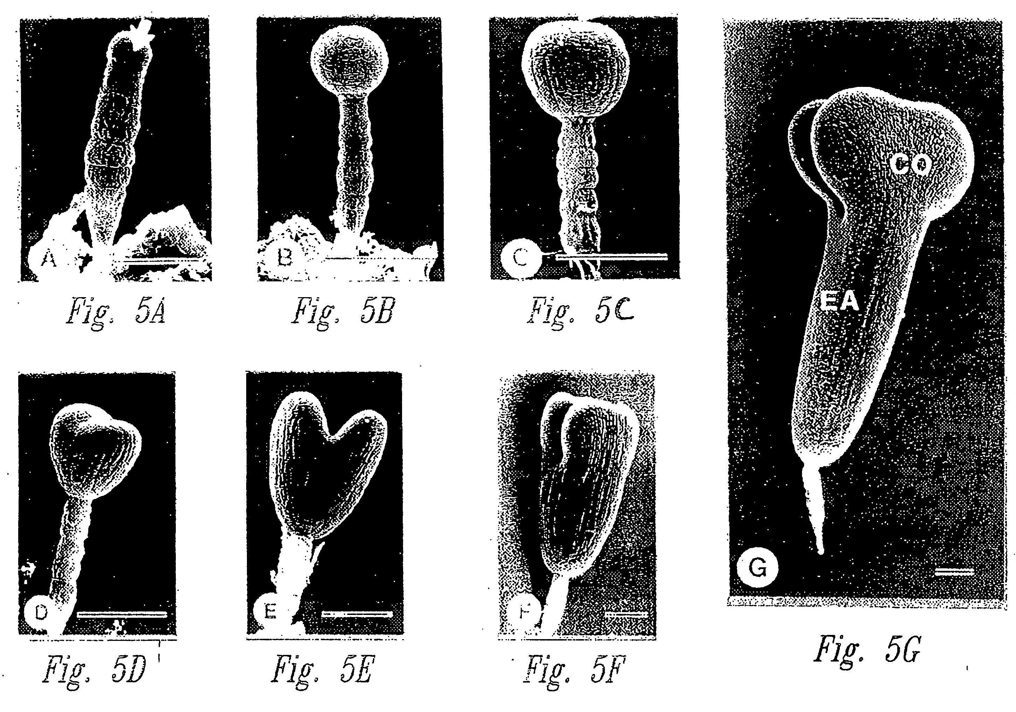

Figures 5A-5G are scanning electron micrographs showing the morphology of developing zygotic embryos of *Brassica napus* cv. Topas dissected from ovules. Figure 5A shows a proembryo at octant stage. Transverse division of quadrant cells delineates the O' boundary (arrow) - this 'line' demarcates the future upper axis that will give rise to shoot apex and cotyledons and lower portion of axis that will develop into hypocotyl and root apex. Scale bar = 50µm. Figure 5B shows an early globular stage embryo. Figure 5C shows an embryo (8 DPA) at transition from globular to heart-shaped stage showing flattened apical region of the embryo proper (arrow). Figure 5D shows a heart-shaped embryo (9-10 DPA), and Figure 5E shows a late heart-shaped embryo. Figure 5F shows a torpedo stage embryo (11 DPA), and Figure 5G shows a linear cotyledon stage embryo with cotyledons (CO) and embryo axis (EA). Scale bars (B-G) = 100 µm.

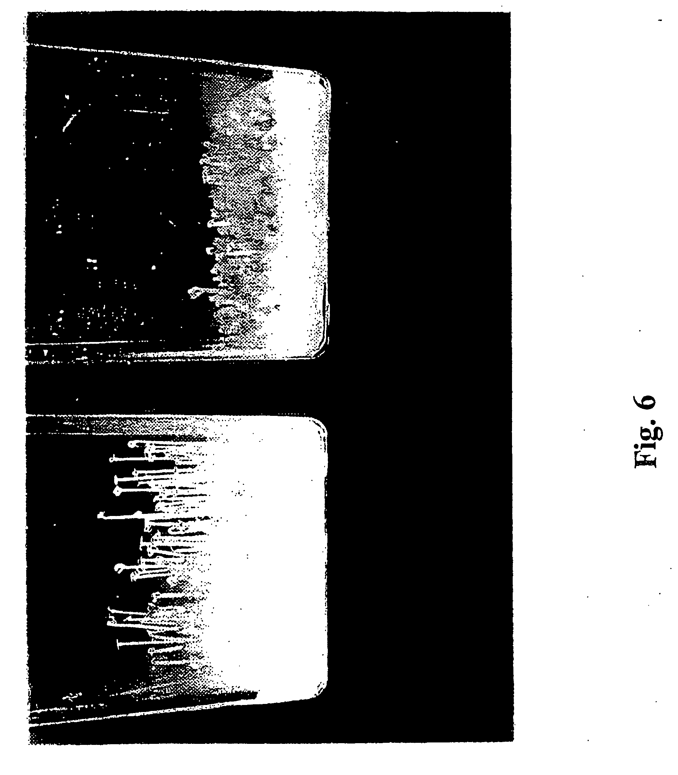

Figure 6 is a photograph of plantlets of *Brassica napus* developed from three week old MD embryos on hormone-free germination medium. The plantlets on the left were developed from embryos grown on PEG-containing medium and those on the right were grown on sucrose-containing medium.

### DETAILED DESCRIPTION OF THE INVENTION

### DEFINITIONS

Prior to setting forth the invention, it may be helpful to an understanding thereof to first set forth definitions of certain terms that will be used hereinafter.

"Plant embryo" refers to a plant structure possessing or capable of developing a root and shoot meristem. Plant embryos may be microspore-derived, somatic, or zygotic.

"Metabolizable carbon source" refers to a carbon-based energy source which is utilized by the plant embryo. Representative examples of metabolizable carbon sources include readily metabolizable substrates (*i*.*e*., substrates such as fructose, glucose, maltose myoinositol, sucrose, and other sugars that are metabolized quickly), as well as poorly metabolizable substrates such as mannitol, sorbitol.

"Osmoticum" refers to compounds which alter the water balance within a cell. Osmotica may be either permeating (*i*.*e*., plasmolysing) or non-permeating (*i*.*e*., non-plasmolysing). Although the ability of an osmoticum to permeate a cell wall depends on the type of cell being treated, generally, non-permeating osmoticum has a molecular size of greater than about 30 Angstroms (*see generally* Carpita et al., *Science 205*:1144-1147, 1979).

"Water potential" refers to the tendency of water to move into or out of a cell. For example, where water stress is effected by means of osmotica, water potential may be readily determined from either the culture medium or from the embryo cell sap utilizing a vapor pressure osmometer (measured in units of mmol/kg; mOs/kg or MPa), or, for many compounds, calculated based upon the concentrations of solute and solution. As a general rule, tissue water potential and osmotic potential can be expressed in Pascals, Newtons per square meter, Barr, or millimoles per kilogram. One MPa corresponds to 403.877 mmol/kg, therefore 1 mmol/kg corresponds to 0.002476 MPa. Tissue water potentials by convention are often negative, but can be given as either negative or positive values. Solution osmolalities are often expressed as positive values.

"Water stress" refers to conditions wherein the lack of moisture (water) inside a cell (*i*.*e*., reduced cellular turgor) stresses cellular processes. Water stress may be caused by environmental conditions (*e*.*g*., relative humidity in the range of about 5% to about 100%), water stressing agents (such as, for example, osmotica or gels), or any other means simulating natural drought stress.

As noted above, the present invention is directed towards methods for generating plant embryos that closely resemble embryos which have undergone natural development. Surprisingly, it has been found that it is possible to uncouple an embryo's osmotic requirements from nutritional requirements. Thus, the methods provided herein recite the step of culturing plant embryos in medium comprising a metabolizable carbon source of less than two percent, under conditions and for a time sufficient to promote embryo development. When cultured under such conditions (along with, in certain variants, water stressing), the immature embryos are well-suited for further maturation to yield fully mature embryos containing levels of storage reserves similar to mature zygotic embryos.

Plant embryos may generally be isolated or generated from any of a variety of plant species. Suitable donor plants include monocots such as the cereals and grasses, dicots such as *Brassica* species, *Hevea basiliensis* (rubber trees), aspens, poplars, oaks, walnuts, cotton, alders, and various species of eucalyptus, and various species of gymnosperms (*e.g.*, conifers such as pines, spruces, douglas fir, larch, hemlock, yew and cedar).

A wide variety of methods may be utilized to generate embryos utilizing, for example, somatic embryogenesis, or, microspore cultures (*see, e*.*g*., Plant Tissue Culture Concepts and Laboratory Exercises, Ed. R.N. Trigiano and D.J. Gray, Publ. CRC Press, Boca Raton, 1996; Plant Cell, Tissue and Organ Culture: Fundamental Methods, ed. O.L. Gamborg and G.C. Phillips, Spmger, NY, 1995).

For example, within one representative procedure for *Brassica napus*, buds are first macerated in a sucrose-containing medium (such as half-strength B5 washing medium (Gamborg et al., *Exp. Cell Res. 50:151-158,* 1968), and the crude suspension is filtered using, for example, 44µm Nytex nylon mesh. The filtrate is then subjected to centrifugation and washing steps using a sucrose-containing medium such as half-strength B5.

To prepare embryos, the pellet containing the microspores (and a small amount of washing medium) is resuspended in PEG-containing medium (*e*.*g*., half-strength NLN containing PEG). PEG is a neutral polymer, highly soluble in water and nontoxic. In solution, PEG creates a more negative water potential, facilitating a water-restricted environment (Rains, *in:* Jones et al. (eds) *Plants under stress*, pp 181-196, 1989 (Cambridge University Press, Cambridge, MA)). The PEG-containing medium preferably contains high molecular weight PEG *(i.e.,* a molecular weight ranging from about 1,000 to 35,000). While the precise molecular weight of the PEG is not critical, the molecular weight should be high enough to substantially prevent penetration of cell walls. In general, a molecular weight of greater than about 1000 is sufficient, and a range of about 1000 to about 8000 is preferred *(see* Carpita et al., *Science 205:* 1144-1147, 1979; Rains, *in:* Jones et al. (eds) *Plants under stress,* pp 181-196, 1989 (Cambridge University Press, Cambridge, MA)). PEG in a variety of molecular weights is readily available from commercial suppliers such as Fluka Chemika, or, Sigma, St. Louis.

The amount of PEG present in the medium generally ranges from about 1% to about 30% (w/v), typically from about 5% to 25% (w/v) and preferably from about 10% to 25% (w/v). The optimal level of PEG may depend, in part, on a variety of factors including the molecular weight of the PEG and the donor plant species, and may be readily determined by those of ordinary skill in the art by evaluating the yield obtained at a series of concentrations. In general, the level of PEG is chosen to provide a water stress similar to or greater than that created by the sucrose in conventional media, but depends in part on the concurrent utilization of other means of water stressing. The potential of a medium may generally be determined using well-known methods, such as by utilizing a vapor-pressure osmometer. It has been found, within the context of the present invention, that one preferable concentration of PEG 4000 for generating embryos form *Brassica napus* microspores is about 22% - 25% w/v.

The concentration of the metabolizable carbon source in the medium should generally range from about 0.001% to about 2%, typically from about 0.5% to about 0.75%, and preferably from about 0.08% to about 0.2% (w/v). Use of very low levels of the metabolizable carbon source is particularly preferred when media is exchanged regularly. In addition, when a poorly metabolized carbon source is utilized, generally higher concentrations are preferred.

The medium may optionally contain small amounts of other carbohydrates, either singly or in combination (see generally Plant Tissue Culture Concepts and Laboratory Exercises, Ed. R.N. Trigiano and D.J. Gray, Publ. CRC Press, Boca Raton, 1996; Plant Cell, Tissue and Organ Culture: Fundamental Methods, ed. O.L. Gamborg and G.C. Phillips, Sprnger, NY, 1995).

After culturing, the embryo suspension may be treated in a variety of manners, depending on the species of plant *(see, e.g.,* U.S. Patent No. 5,464,769; see also Plant Tissue Culture Concepts and Laboratory Exercises and Plant Cell, Tissue and Organ Culture, *supra*).

For *Brassica napus*, the microspore suspension may then be plated onto petri dishes with a plating density of, within one embodiment, about 40,000 microspores/ml. While incubation conditions may vary, the following series of incubations has been found, within the context of the present invention, to be effective for *Brassica napus* microspores: a short incubation at 35°C (*e*.*g*., one hour), followed by a longer incubation at 33°C (*e*.*g*., 14-18 hours) and a two-week incubation at 24°C on a shaker and in the dark. A gradual dilution of the PEG medium (by, for example, adding half-strength NLN medium without sucrose or PEG) over the first approximately 8 days and resulting in a final concentration of about 22% PEG may be beneficial. After 14 days at 24°C in the dark, embryos will have developed. Some of these embryos may be 1-3mm in length, reaching the cotyledonary stage, but most are generally smaller, still in the globular, heart-shaped or torpedo stages.

Plates containing the embryos may then be placed under light at 24°C for an additional week. Once placed under light, the embryos generally increase in size and become green quickly. Within one week under light, cotyledons are typically well-developed, often widely opened and dark green, and the embryos are morphologically similar to zygotic embryos dissected from ovules *(see* Figures 2A-2C). The three-week embryos may then be rinsed and transferred to culture containers containing a suitable medium, such as half-strength B5 medium containing 1% sucrose and no growth regulators. The conversion frequency is the percentage of MD embryos that develop roots, true leaves and a green color within 3-4 weeks.

Using routine experimentation, the above procedure may be further optimized for *Brassica* species and may be adapted to other plant species as well. To optimize growth conditions, embryo morphology may be monitored during growth at any of a variety of developmental stages using well-known scanning electron microscopy (SEM) techniques. SEM analyses provide a unique opportunity for three-dimensional observations that allow visualization of surface-associated changes occurring during earlier embryogenic stages. Briefly, embryos may be fixed in, for example, gluteraldehyde and osmium tetroxide, dried, sputter coated with gold and examined in a scanning electron microscope. Such analyses may be performed on embryos during development under different conditions, thereby facilitating optimization of growth conditions.

As noted above, embryos prepared by the above method have the advantage of more closely resembling zygotic embryos than embryos prepared by conventional means. It will be readily apparent that this advantage provides benefits for genetic manipulation and developmental studies, and for commercial procedures involving the generation of large numbers of plants. While *Brassica napus* is employed herein as an example, the application of the present methods to a broad range of plant species is contemplated. Accordingly the present invention encompasses the generation of embryos from other *Brassica* species, such as self-incompatible *Brassica rapa*, as well as more distantly related plants, including for example, monocots such as the cereals and grasses, dicots such as *Brassica* species, *Hevea basiliensis* (rubber trees), aspens, poplars, oaks, walnuts, cotton, alders, various species of eucalyptus, and various species of gymnosperms (*e.g.*, conifers such as pines, spruces, douglas fir, larch, hemlock, yew and cedar).

The following examples are offered by way of illustration and not by way of limitation.

### EXAMPLES

### EXAMPLE 1

### Preparation of Embryos From Brassica napus

This example illustrates the growth of donor plants, the isolation of microspores and the generation of embryos from the representative species *Brassica napus*.

### Growth of Donor Plants

Plants of *Brassica napus* L. cv. Topas line 4079 were grown in a growth chamber with a 20°/15°C day/night temperature regime and 16 hour illumination provided by VHO (very high output) Sylvania cool white flourescent lamps. Prior to bolting, the temperature was lowered to 10°/5°C (day/night) and plants were maintained at this regime providing water and nutrients twice per week with 0.35 g/L of 15-15-18 (N-P-K) nutrient solution (Ferrie and Keller, *in:* Gamborg and Phillips (eds) *Plant Cell, Tissue and Organ Culture, Fundamental methods,* pp 155-164, 1993 (Springer Verlag)). Separate sets of plants were grown at 22°/15°C day/night temperature regime and 16/8 hour photoperiod, and hand-pollinated flowers were tagged to indicate days after anthesis (DPA). Immature zygotic embryos were dissected from developing ovules (14 and 15 DPA) and used for comparison with MD embryos.

### Isolation of Microspores

A mixed population consisting of microspores from mid- and late-uninucleate stages to young bicellular pollen was isolated from the flower buds using a protocol modified from that described by Ferrie and Keller, *in:* Gamborg and Phillips (eds) *Plant Cell, Tissue and Organ Culture, Fundamental methods,* pp 155-164, 1993 (Springer Verlag). For each treatment, twelve surface-sterilized buds (3.6-4.2 mm in length) were macerated in 5 ml half-strength B5 washing medium (Gamborg et al., *Exp. Cell Res. 50*: 151-158, 1968) containing 13% sucrose (w/v) in 50 ml beakers. The crude suspension was filtered through 44 µm Nytex nylon mesh, both beakers and meshes were rinsed, and a total of 20 ml was collected into 50 ml centrifuge tubes and centrifuged at 150 x g for 3 minutes. The pellets were resuspended in 5 ml of washing medium, the washing procedure was repeated twice, and the final supernatant was carefully pipetted out, leaving the pellet in approximately 0.3-0.4 ml of washing medium. Final resuspension was done by adding filter-sterilized half-strength NLN medium (Lichter, *Z. Pflanzenphysiol. 105*:427-434, 1982) containing 13% (w/v) sucrose as the control treatment; in the other two treatments, ½ NLN sucrose-free medium with 8% (w/v) mannitol or 25% (w/v) PEG 4000 (Fluka Chemika) was added, respectively. Therefore, the ½ NLN-mannitol and -PEG media contained only about 0.08%-0.1% sucrose, remaining from the washing medium.

Aliquots of 10 ml of microspore suspension were plated into 10 x 15 mm sterile petri dishes, with an approximate plating density of 40,000 microspores/ml. Plates were first incubated at 35°C for one hour followed by 33°C for the next 14-18 hours and then placed on a shaker (50 rpm) in the dark at 24°C for 14 days. During the first 8 days in culture, PEG and mannitol media were gradually diluted by adding to the plates an adequate volume of a simple ½ NLN medium, containing no sucrose, mannitol, or PEG, to give a final concentration of approximately 22% PEG and 7% mannitol. The concentration of sucrose in the control treatment was lowered the same way to a final concentration of 11 %. After two weeks, plates with MD embryos were placed under light (16/8 hours photoperiod) at 24°C for an additional week. Experiments dealing with mannitol as osmoticum were repeated three times with five replicates, while experiments dealing with PEG and sucrose as a control were repeated at least ten times with a minimum of three replicates.

### Results and Discussion

In order to meet specific osmotic requirements for microspore embryogenesis and to avoid potential abundant sugar uptake by embryos, mannitol and PEG were applied as alternative osmotic agents in concentrations that provided water potential similar to that created by sucrose. Therefore, sucrose as a mere metabolizable carbon source was present in the medium only in a residual amount (approximately 0.08-0.1%).

Induction of MD embryos depended on the type of osmoticum used: microspores cultured on sucrose and PEG media developed into embryos within two weeks, while mannitol-cultured microspores yielded no embryos. After two weeks in culture, some of the PEG embryos were about 1-3 mm in length, reaching the cotyledonary stage; however, most of them were smaller, still in the globular, heart-shaped or torpedo stages (Figure 1A). When placed under light, these embryos quickly became green (Figures 1A and 1C) and further increased in size, due to elongation of the embryo axis and further differentiation of cotyledons. An assessment of frequency of embryo formation was not attempted, due to the technical difficulty of counting PEG embryos of such a small size. Nevertheless, the number of microspores induced to undergo embryogenic development appeared comparable to that of the sucrose control (Figure 1A), based on visual observation of microspores and developing embryos with the inverted light microscope.

Since the size of MD embryos rather than their total number was affected on PEG medium, PEG indeed provides a proper osmotic environment for induction of microspore embryogenesis in *Brassica napus*. The smaller size of PEG embryos compared to sucrose embryos is likely due to restricted carbon supply, as expected given the minute amount of metabolizable carbon provided in the medium. Interestingly, the high viscosity of the PEG solution and restricted water supply did not affect early microspore development upon inductive heat shock treatment, which is considered to be a sensitive stage. In contrast, mannitol, a solute generally considered to penetrate cells passively and very slowly (Cram, *Physiol. Plant 61*:396-404, 1984), had a negative effect on induction of microspore embryogenesis. Microspores in mannitol solution became swollen after heat shock treatment, but no divisions were observed and microspores were presumably dead within a week in culture.

After transfer to solid medium, MD embryos first developed elongated hypocotyls and radicles, then formed roots, and some subsequently formed the first true leaves. PEG embryos converted into plantlets with dark green cotyledons, very elongated hypocotyls and well-developed roots. Sucrose embryos also had well-developed roots, but the cotyledons were still pale green and hypocotyls were short, often thick (hyperhydrated) and aberrant. These hypocotyls later produced numerous secondary embryos.

Under restricted sugar and water supply, the size of the PEG embryos was reduced when compared to sucrose embryos, but they were morphologically very similar to immature zygotic embryos dissected from ovules at 14 and 15 DPA (Figures 1E and 1F). Three-week-old PEG embryos had well-developed cotyledons, often widely opened and dark green, suggesting possible photosynthetic activity (Figure 1F). MD embryos on sucrose medium, when compared to both zygotic and PEG embryos, were pale green, had elongated embryo axes and relatively small underdeveloped cotyledons. These features have often been reported in *Brassica napus* MD embryos (Rahman, Microspore-derived embryos of *Brassica napus* L.: Stress tolerance and embryo development, Ph.D. Thesis, University of Calgary, 1993; Hause et al., *Bot. Acta 107*: 407-415, 1994; Yeung et al., *Int. J. Plant Sci. 157*:27-39, 1996).

Plantlets were developed from three week old MD embryos on hormone-free medium. As shown in Figure 6, those that were developed from embryos grown on PEG-containing medium (left) were larger than those grown on sucrose-containing medium (right).

These results demonstrate that sucrose as an osmoticum can be replaced with high molecular weight PEG in microspore embryogenesis of *Brassica napus.* The amount of metabolizable carbon source required for MD embryo induction and formation appears to be substantially less than reported previously. Morphologically, PEG embryos are strikingly similar to immature zygotic embryos dissected from ovules. Thus, embryogenesis is more effective when PEG is used as an osmoticum, with only a minute quantity of metabolizable carbon source in the medium.

### EXAMPLE 2

### Comparative Morphological and Histological Analyses of Zygotic and Microspore-Derived Embryos

This Example illustrates the investigation of morphology of MD embryos grown on sucrose and PEG osmoticum.

Morphological characteristics of microspore- and pollen-induced embryos of *Brassica napus* L. cv. Topas were investigated by scanning electron microscopy (SEM). Embryos were induced from microspores at the late uninucleate stage as well as from young bicellular pollen, when subjected to the heat shock treatment - a requirement for the 'switch' from microspore/pollen development to embryo formation *in vitro.* Two different types of osmoticum were applied in liquid culture medium as described in Example 1: sucrose (13% w/v) as a permeating osmoticum and polyethylene glycol (PEG) as a non-permeating osmoticum (22-25% w/v).

Induction and subsequent development of embryos occurred on both osmotica; however, striking differences in morphology were revealed by SEM. In sucrose medium, embryos were large but poorly differentiated, with small cotyledons. In contrast, PEG-grown embryos were small, and developed a single file of cells resembling suspensors upon break down of the exine. These embryos exhibited bilateral symmetry during the transition from globular to heart-shaped in a similar fashion to zygotic embryos *in ovulo.* Light microscopy of cotyledon stage embryos grown on sucrose showed abundant starch grains with no protein bodies; PEG embryos had considerably less starch and some protein bodies were present (Figures 3A-3C).

### EXAMPLE 3

### Morphological Analysis of Microspore-Derived Embryos

This example illustrates the use of scanning electron microscopy to examine morphological changes during the development of microspore-derived embryos grown using reduced levels of sucrose, in a PEG-mediated low water potential environment.

### Growth of Donor Plants

Plants of *Brassica napus* L. cv. Topas line 4079 were grown in a growth chamber with a 20°/15°C day/night temperature regime and 16 hour illumination provided by VHO (very high output) Sylvania cool white flourescent lamps. Prior to bolting, temperature was lowered to 10°/5°C (day/night) and plants were maintained at this regime providing water and nutrients twice per week with 0.35 g/L of 15-15-18 (N-P-K) nutrient solution (Ferrie and Keller, *in*: Gamborg and Phillips (eds) *Plant Cell, Tissue and Organ Culture, Fundamental methods,* pp 155-164, 1993 (Springer Verlag)). Separate sets of plants were grown at 22°/15°C day/night temperature regime and 16/8 hour photoperiod and hand-pollinated flowers were tagged to indicate days after anthesis (DPA). Developing ovules from main inflorescences were collected for dissection and further processing of zygotic embryos for SEM.

### Isolation of Microspores and Immature Pollen

Microspores at late unicelullar stage and young bicellular pollen were isolated from the flower buds using a protocol for *Biassica napus*, modified from that described by Ferrie and Keller, *in:* Gamborg and Phillips (eds) *Plant Cell, Tissue and Organ Culture, Fundamental methods*, pp 155-164, 1993 (Springer Verlag). Twelve surface-sterilized buds (3.6-4.2 mm in length) were macerated in a 50 ml beaker with 5 ml half-strength B5 washing medium (Gamborg et al., *Exp. Cell Res. 50*:151-158, 1968) containing 13% sucrose (w/v). The crude suspension was filtered through 44 µm nylon mesh and centrifuged at 150 x g for 3 minutes, and the pellet was resuspended in 5 ml of washing medium. The washing procedure was repeated twice, and the final supernatant was carefully pipetted out, leaving the pellet in approximately 0.3-0.4 ml of washing medium. Final resuspension was done by adding filter-sterilized half-strength NLN medium (Lichter, *Z. Pflanzenphysiol 105*:427-434, 1982) containing either 13% sucrose (w/v) as a control or 25% (w/v) polyethylene glycol 4000 (Fluka Chemika). Therefore, the final ½ NLN-PEG medium contained only about 0.08%-0.1% sucrose, remaining from the washing medium.

Aliquots of 10 ml of the microspore suspension were plated into 10x15 mm sterile petri dishes, with an approximate plating density of 40,000 microspores/ml. Plates were first incubated at 35°C for one hour followed by 33°C for the next 14-18 hours and then placed on a shaker (50 rpm) in the dark at 24°C for 14 days. During the first 8 days in culture, PEG medium was gradually diluted by adding an adequate volume of ½ NLN medium, containing no sucrose or PEG, to give a final concentration of 22% PEG. The concentration of sucrose medium was lowered in the same way to a final concentration of 11%. After two weeks, plates with MD embryos were placed under the light (16/8 hours photoperiod) for an additional week. Three-week-old cotyledonary embryos were sieved through 500 µm nylon mesh screen, washed and transferred to solid ½ B5 medium containing 0.7% agar, 1% sucrose and no growth regulators for another two to three weeks. Conversion frequency (%) of sucrose- and PEG-cultured MD embryos was recorded based on presence of roots, appearance of first true leaves and green color of plantlets, with three replicates for each treatment.

In a separate set of experiments, individual buds of a) 3.6 mm and b) 4.2 mm in length were picked from two month-old plants and used for isolation of the late unicellular microspores and young bicellular pollen respectively. Sterile buds were macerated in a 10 ml beaker and filtered through nylon mesh into 15 ml polypropylene tubes (Falcon 2099), centrifuged as described and washed three times. After the last centrifugation, the supernatant was carefully pipetted out, leaving approximately 0.1 ml of ½ B5 washing medium in the tubes prior to final resuspension in 4.8 ml of ½ NLN medium containing sucrose and PEG. Microspore suspensions were plated in 35 x 10 mm Falcon 1008 petri dishes (1.2 ml/plate). Plating density varied and was estimated to be approximately 20,000 microspores/ml. To determine the developmental stage of microspores, small samples of a) and b) treatments were collected after the first centrifugation in 0.5 ml Eppendorf tubes and stained with aqueous DAPI solution, final concentration 2µg/ml in 1% Triton X 100, (Coleman and Goff, *Stain Technol. 60*:145-154, 1985). Microspores were gently spun and immediately observed with a Zeiss Axioplan epifluorescence microscope equipped with HBO 50 W mercury lamp and standard filter set for DAPI.

### Scanning Electron Microscopy

Samples from microspore cultures were placed in 0.5 ml Eppendorf tubes under sterile conditions at 6, 9 and 14 days after initiation. Microspores and embryos cultured on sucrose medium were fixed sequentially in 1% and 3% glutaraldehyde in 0.05 M phosphate buffer, prepared in 10% sucrose. Embryos cultured on PEG medium were first washed for 3 minutes in half-strength NLN medium containing 8% mannitol, then also fixed sequentially in 1% and 3% glutaraldehyde in 0.05M phosphate buffer made with 8% mannitol. Samples were postfixed overnight in 1% osmium tetroxide, washed in distilled water 3-4 times and dehydrated in a graded acetone series (Fowke et al. 1994). For SEM, critical point drying (CPD) of extremely small samples (50-300µm) was carried out in specially-adapted beam capsule baskets, placed directly in the trough of the critical point dryer. These baskets were made by cutting off the sloped portion of the beam embedding capsules (J.B. EM Services Inc.) so that lids would fit both ends. Each lid was punched out with a hole-punch and fitted with 50µm Nytex nylon mesh discs, 10 mm in diameter. Up to six of these baskets can stand upright in the trough of the Polaron E3000 Critical Point Dryer. After CPD, samples were mounted on SEM stubs by using double-sided tape, sputter coated with gold in an Edwards Sputter Coater, Model S150B and examined in a Philips 505 Scanning Electron Microscope at 30 kV. Images were recorded on Polaroid 665 film.

### Results

### In vitro development of microspore-derived embryos on sucrose and PEG medium

During the first six days in culture, the enlargement of microspores proceeded in a very similar manner on both media. After heat shock treatment, swollen microspores gradually increased in size, due to first cell divisions while still within the original pollen wall, eventually reaching 35-50 µm in diameter before the final breakdown of the pollen wall occurred (about day 5 in culture). Three different kinds of morphological changes were observed during this period. In type I, swollen microspores became globular, their original tricolpate structure became less distinct as the furrows flattened, the pollen wall stretched uniformly over the entire surface, and expanding ridges of reticulate exine sometimes broke due to the mechanical stress. There is little evidence, however, of how the final breakdown of pollen wall occurred, and more important, whether these microspores continued development beyond the first six days in culture. In another, more common type (type II), enlargement of microspores caused a split in the pollen wall along all three colpae; an increase in volume of microspores was localized in these regions, while reticulate exine ornamentation changed very little. After five days in culture, the growing cell cluster usually broke out along one of the ruptured furrows, while remnants of the pollen wall adhered on one side of the globular proembryo, later visible as a pocket-like structure (Figure 4A). In type III, all three colpae cracked as the growing microspore expanded; however, instead of formation of a compact multicellular cluster, only three (or more) enlarged and loosened cells emerged, causing the pollen wall to break down along one of the ruptured furrows. These probably give rise to non-embryogenic clusters of loosely connected cells with no distinct morphological pattern, often seen during the first several days in culture. It is noteworthy that the final breakdown of pollen wall appeared to be a critical point during the early development of microspores in vitro; many enlarging microspores collapsed upon breakdown of the original pollen wall. This was commonly observed on both media.

Once the globular mass broke out of the pollen wall, a substantial enlargement of the proembryo took place prior to the first evidence of transition from radial to bilateral symmetry that became evident as MD embryos became slightly oval. Such enlargement was more advanced in MD embryos on sucrose medium; here, proembryos reached 150-250 µm in length or larger before the first sign of globular-to-heart-shaped transition occurred, usually about day 8 (Figure 4B). PEG-cultured proembryos were about 100-150 µm in diameter when a flattened apical region became slightly concave, and emergence of cotyledonary lobes on the oval-shaped embryos became more distinct (Figure 4G). The appearance of small appendages at the basal region of MD embryos was observed very often at this stage on both sucrose and PEG embryos. These structures, resembling suspensors of zygotic embryos, emerged as a result of transverse cell divisions that occurred in a particular peripheral cell of the globular proembryo upon the breakdown of the pollen wall.

Emergence and outgrowth of two cotyledonary lobes and elongation of the embryo characterized the heart-shaped stage of embryo development. Sucrose embryos were approximately 300 µm in length at this stage, while PEG embryos were in most cases half that size, about 150 µm (Figures 4C and 4H). At this stage, a range of different shapes and sizes of suspensor were observed, from single-filed filamentous structures to small, multicellular protrusions, irregular in shape (Figures 4B and 4H). However, not all the MD embryos exhibited these structures. Also, there was variability among experiments. In some, the majority of embryos had suspensors while in others only a few suspensors were found.

Subsequently, emerging cotyledons expanded rapidly and the embryo axis continued to elongate giving rise to early torpedo-shaped embryos. Sucrose-grown embryos reached a size of about 400 µm or larger, primarily as a result of axis elongation, whereas cotyledons were rather short and undeveloped - their expansion was slow and delayed. PEG embryos at the torpedo stage were about 250 µm - 300 µm in length, had well-expanded cotyledons as well as embryo axis (Figure 4I).

Two-week-old cultures already contained some cotyledonary stage embryos, while the bulk of the embryos were still in the torpedo or heart-shaped stages. Also, there were globular clumps of non-embryogenic undifferentiated cells. Early cotyledonary stage embryos were approximately 2-4 mm in length when grown on sucrose and only 0.7 mm to 2 mm when grown on PEG (Figures 4E and 4K). Sucrose embryos had rather small, underdeveloped cotyledons and a disproportionately elongated embryonic axis. Very often radicles showed some sloughing of epidermal cells, which progressed toward the apical region as the embryos become older. In contrast, PEG embryos had two well-developed cotyledons, proportionate embryo axis and a smooth epidermal surface.

Once placed under light, all MD embryos further increased in size and became green. PEG embryos had widely opened cotyledons, dark green and possibly photosynthetically active, whereas sucrose embryos had small, pale green cotyledons and very elongated hypocotyls.

### Pollen wall fragments attached to microspore- and pollen-induced embryos

Suspensors were observed at about 9 days in culture on embryos in both media, however, they were not seen on all MD embryos at the globular and heart-shaped stages. Some MD embryos had a rather rudimentary protrusion at the future root pole, or instead, had only remnants of the original pollen wall adhering on their surface. These remnants were in most cases located at the future root pole region of the developing embryo. These fragments were easily detected by SEM because of their reticulate exine pattern and were observed throughout the development of MD embryos. They were conspicuous on smaller embryos (globular and heart-shape) and less noticeable on large cotyledonary stage embryos, due to the large size of the embryos compared to the minute size of these fragments. Furthermore, fragments of the original pollen wall were consistently observed at the tips of the suspensors, regardless of their shapes or sizes. These observations raise the question of the polarity of microspore-derived embryos, which appeared to be established at a very early stage of development, while the microspore was still enclosed within its wall.

In order to test if there were any differences regarding this phenomenon between embryos originating from a population of microspores at the late uninucleate stage and immature bicellular pollen, a separate set of experiments involving individual buds of two different sizes was carried out.

Freshly isolated microspores from small buds (3.6 mm) were mostly in late uninucleate stage, as revealed by DAPI; however, there was a small percentage ( 1%-5%) of microspores in division and an early bicellular stage. Large buds (4.2 mm) consisted entirely of immature pollen at the early bicellular (generative cell in close proximity to intine) and late bicellular (generative cell, bounded by its plasma membrane, has migrated into interior of vegetative cell) stages (Telmer et al., *Protoplasma 172:* 154-165, 1993). Two weeks after isolation, embryos were obtained from both treatments, regardless of the osmoticum used. Fragments of reticulate exine were frequently observed in both microspore-induced and young pollen-induced embryos; suspensors were also found in both groups.

### Morphology of zygotic embryos dissected from developing ovules

In this study, zygotic embryos at transition stage of development (Figures 5A-5G) were the youngest embryos successfully obtained from dissected ovules (7 DPA). The embryo proper was about 80 µm in size, and distinct derivatives of the hypophysis (suspensor distal cell) could be seen. Flattening of the embryo proper followed by a slight widening of the terminal region of the embryo proper was the first indication of a transition from radial to bilateral symmetry (Figure 5C). Further outgrowth of distal lobes and apical-basal elongation resulted in formation of heart-shaped embryos with the two major embryonic organ systems being well-defined: cotyledons and embryo axis (9-10 DPA). Considerable elongation of cotyledons and embryo axis gave rise to torpedo-shaped embryos (Figure 5F) of approximately 400 µm in length. As the rapid elongation of the embryo progressed, cotyledons soon became flattened and leaf-like, while a linear embryo axis, consisting of shoot apex, hypocotyl and root apex, reached about 0.70 µm in length, with still distinct suspensor (Figure 5G). Further stages of embryo development were related to the embryo maturation period, during which cotyledons and embryo axis fully expand folding back and gradually filling the entire ovule.

### Discussion

### Morphology of MD embryos induced on sucrose and PEG-mediated low water potential medium

In order to meet specific osmotic requirements for microspore embryogenesis, and to avoid abundant sugar uptake by embryos, PEG as an alternative osmotic agent was applied in concentrations that provide a water potential similar to that created by sucrose. In general, embryos produced on PEG medium proceeded in their development from microspores to cotyledon stage embryos in a similar manner to those on sucrose medium. Morphological changes described while the microspores were still within their original pollen wall were observed on both osmotica. We were not able to detect any difference between these two systems during this early period.

After the first week in culture, as the globular proembryos enlarged, the differences between the two became obvious. Suspensors, small protrusions at the future basal pole, appeared on both sucrose and PEG embryos. Yet PEG embryos frequently had filamentous single-file suspensors that often exceeded the length of the embryo itself. Another striking difference between the PEG embryos and sucrose embryos was the size of the cotyledons. Under limited sugar and water supply in PEG medium, the size of the whole embryo was reduced; however, morphologically, these embryos appeared superior to those cultured on sucrose. In earlier experiments which included higher sucrose levels (4% and 6.25%) in NLN-PEG medium, embryos obtained were comparable in length and cotyledon size to those cultured on sucrose alone (data not shown). This suggests that the limited amount of sucrose available in the medium, rather than just the PEG-mediated water restricted environment itself, is important for triggering such morphological responses. In PEG embryos, differentiation of cotyledons proceeded normally, from emerging distal lobes at the heart stage to fully expanded, dark green leafy structures of three-week-old embryos.

### Morphological differences between zygotic embryos developed in ovulo and MD embryos induced on PEG medium

Overall, many similarities in morphological differentiation of zygotic embryos developed *in ovulo* and MD embryos induced on PEG medium were observed in this study. However, significant differences that appeared in pattern formation need to be outlined. The zygotic embryo begins as a polarized single cell, the zygote, whose first asymmetrical transverse division gives rise to two structurally different cells: the apical and basal cells. The small apical cell divides in a series of precisely defined, predictable cell divisions forming most of the embryo proper, whereas the basal cell continues transverse divisions, producing a filamentous single-filed suspensor with its uppermost cell - the hypophysis. This cell becomes incorporated into the body of the proembryo and its derivatives contribute, in part, to root apex formation (Tykarska, *Acta Societatis Botanicorum Poloniae 45:3-16,* 1976; Tykarska, *Acta Societatis Botanicorum Poloniae* 49:391-421, 1979). Therefore, a strictly determined cell division pattern exists in zygotic embryogenesis of *Brassica napus* resulting in a linear cotyledon stage embryo that has three spatial domains: apical domain (cotyledons, shoot apex and upper hypocotyl), central domain (bulk of the embryo axis), and basal domain (root apex) (Jürgens et al., *Development* (suppl) *1*:27-38, 1991, West and Harada, *Plant Cell 5*:136-1369, 1993). The boundary between the apical domain and other two domains - transverse O' line - appears as early as at the octant stage of the proembryo (Tykarska, *Acta Societatis Botanicorum Poloniae 45*:3-16, 1976; Tykarska, *Acta Societatis Botanicorum Poloniae 49*:391-421, 1979).

In contrast, such a precise cell division pattern was missing in microspore embryos (Yeung et al., *Int. J. Plant Sci. 157*:27-39, 1996). Our SEM study indicates similar findings. Early embryo development from microspore and young bicellular pollen varied considerably; suspensor formation was delayed and when formed, its size and shape differed; no hypophysis nor its derivatives were located in MD embryos, and O' boundary was not detectable at any of the stages described. Nevertheless, the final cotyledon stage embryo, with all three domains evident (Figure 4J) is amazingly similar to its zygotic counterpart. It seems, therefore, that strictly determined cell divisions occurring during early proembryo development are not crucial for the establishment of three embryonic domains.

From the foregoing it will be appreciated that, although specific embodiments of the invention have been described herein for purposes of illustration, various modifications may be made without deviating from the scope of the invention. Accordingly, the invention is not limited except as by the appended claims.

## Claims

1. A method for culturing embryos of a plant species to maturity in the presence of a metabolizable carbon source comprising selecting the amount of said metabolizable carbon source in the range from about the amount necessary to meet the minimal nutritional requirements for the embryos but no more than about the amount which is necessary to promote normal development of embryos of said plant species, and further comprising water stressing the embryos other than by means of additional amounts of metabolizable carbon sources, to create a water potential of at least 300 mmol/kg.

2. The method of claim 1, wherein the metabolizable carbon source is present in a concentration of less than about 2% w/v.

3. The method of claim 1, wherein the metabolizable carbon source is present in a concentration of less than about 1% w/v.

4. The method of claim 1, wherein the embryos are selected from the class comprising angiosperm embryos.

5. The method of claim 4, wherein the embryos are selected from the class comprising dicotyledonary angiosperm embryos

6. The method of claim 5, wherein the embryos are from the genus *Brassica*.

7. The method of claim 6 wherein the embryos are from the species *napus*.

8. The method of claim 1, wherein the embryos are selected from the class comprising somatic, zygotic, and microspore-derived embryos.

9. The method of claim 8, wherein the embryos are microspore-derived.

10. The method of claim 1 wherein said water potential is at least about 750 mmol/kg.

11. The method of claim 1, wherein the means of water stressing is selected from the class comprising environmental effect, water stressing agents, and combinations thereof.

12. The method of claim 11 wherein the water stressing agent comprises at least one gel.

13. The method of claim 11 wherein the water stressing agent comprises at least one osmoticum.

14. The method of claim 13, wherein the osmoticum is selected from the group comprising non-permeating osmotica.

15. The method of claim 13 wherein the osmoticum has a molecular size of at least about 30 Angstrom units (Å).

16. The method of claim 13, wherein the osmoticum is selected from the group comprising polyalkylene glycols.

17. The method of claim 16 wherein the osmoticum comprises at least one polyalkylene glycol having a minimum molecular weight of about 1,000.

18. The method of claim 11 wherein the environmental water stressing is applied at least in part by relative humidity in the range of 5% - 100%.

19. The method of claim 1, wherein said metabolizable carbon source is selected from the group comprising fructose, glucose, maltose, myo-inositol and sucrose.

20. The method of claim 1, wherein said metabolizable carbon source is selected from the group comprising poorly metabolized carbon sources.

21. The method of claim 20 wherein said poorly metabolized carbon source is a sugar alcohol.

22. The method of claim 20, wherein the concentration of said carbon source is sufficiently low to minimize toxic effects on the embryos.

23. A medium for culturing plant embryos of a plant species to maturity comprising:
(i) a metabolizable carbon source, said carbon source being present in a concentration of up to about 2% w/v, but no more than the amount necessary to promote normal development of embryos of said plant species, and
(ii) a non-permeating osmoticum which is other than an additional amount of metabolizable carbon source, the concentration of said osmoticum being selected to create a water potential of at least 300 mmol/kg.

24. The medium of claim 1, wherein the metabolizable carbon source is selected from the class comprising fructose, glucose, maltose, myo-inositol and sucrose.

25. The medium of claim 1, wherein the concentration of said osmoticum is at least about 7% w/v.

26. The method of claim 1, wherein said embryos comprise gymnosperm embryos.

27. The method of claim 26, wherein said embryos are from a pine species.

## Patentansprüche

1. Verfahren zum Kultivieren von Embryonen einer Pflanzenspezies bis zur Reife in Gegenwart einer metabolisierbaren Kohlenstoffquelle, umfassend das Wählen der Menge dieser metabolisierbaren Kohlenstoffquelle im Bereich von etwa der Menge, die notwendig ist, um die Mindestnährstoffanforderungen für die Embryonen zu erfüllen, aber von nicht mehr als etwa der Menge, die notwendig ist, um die normale Entwicklung von Embryonen dieser Pflanzenspezies zu fördern, und ferner umfassend Ausüben von Wasserstress auf die Embryonen mit anderen Mitteln als durch zusätzliche Mengen an metabolisierbaren Kohlenstoffquellen zur Erzeugung eines Wasserpotentials von wenigstens 300 mmol/kg.

2. Verfahren nach Anspruch 1, wobei die metabolisierbare Kohlenstoffquelle in einer Konzentration von weniger als etwa 2% w/v vorliegt.

3. Verfahren nach Anspruch 1, wobei die metabolisierbare Kohlenstoffquelle in einer Konzentration von weniger als etwa 1% w/v vorliegt.

4. Verfahren nach Anspruch 1, wobei die Embryonen ausgewählt sind aus der Klasse umfassend Embryonen von Angiospermen.

5. Verfahren nach Anspruch 4, wobei die Embryonen ausgewählt sind aus der Klasse umfassend Embryonen von dikotyledonen Angiospermen.

6. Verfahren nach Anspruch 5, wobei die Embryonen von der Gattung *Brassica* sind.

7. Verfahren nach Anspruch 6, wobei die Embryonen von der Spezies *Napus* sind.

8. Verfahren nach Anspruch 1, wobei die Embryonen ausgewählt sind aus der Klasse umfassend somatische, zygotische und von Mikrosporen stammende Embryonen.

9. Verfahren nach Anspruch 8, wobei die Embryonen von Mikrosporen stammen.

10. Verfahren nach Anspruch 1, wobei das Wasserpotential wenigstens etwa 750 mmol/kg beträgt.

11. Verfahren nach Anspruch 1, wobei das Mittel zum Ausüben von Wasserstreß ausgewählt ist aus der Klasse umfassend Umwelteffekte, Wasserstreß verursachende Mittel und Kombinationen davon.

12. Verfahren nach Anspruch 11, wobei das Wasserstreß verursachende Mittel wenigstens ein Gel umfaßt.

13. Verfahren nach Anspruch 11, wobei das Wasserstreß verursachende Mittel wenigstens ein Osmotikum umfaßt.

14. Verfahren nach Anspruch 13, wobei das Osmotikum ausgewählt ist aus der Gruppe umfassend nichtpermeierende Osmotika.

15. Verfahren nach Anspruch 13, wobei das Osmotikum eine Molekülgröße von wenigstens etwa 30 Angström-Einheiten (Å) besitzt.

16. Verfahren nach Anspruch 13, wobei das Osmotikum ausgewählt ist aus der Gruppe umfassend Polyalkylenglykole.

17. Verfahren nach Anspruch 16, wobei das Osmotikum wenigstens ein Polyalkylenglykol mit einem Mindestmolekulargewicht von etwa 1000 umfaßt.

18. Verfahren nach Anspruch 11, wobei das Ausüben von Wasserstreß durch die Umwelt wenigstens teilweise durch relative Feuchte im Bereich von 5% - 100% erfolgt.

19. Verfahren nach Anspruch 1, wobei die metabolisierbare Kohlenstoffquelle ausgewählt ist aus der Gruppe umfassend Fructose, Glucose, Maltose, Myoinositol und Sucrose.

20. Verfahren nach Anspruch 1, wobei die metabolisierbare Kohlenstoffquelle ausgewählt ist aus der Gruppe umfassend schlecht metabolisierte Kohlenstoffquellen.

21. Verfahren nach Anspruch 20, wobei die schlecht metabolisierte Kohlenstoffquelle ein Zuckeralkohol ist.

22. Verfahren nach Anspruch 20, wobei die Konzentration der Kohlenstoffquelle niedrig genug ist, um toxische Effekte auf die Embryonen zu minimieren.

23. Medium zur Kultivierung von Pflanzenembryonen einer Pflanzenspezies bis zur Reife, umfassend:
(i) eine metabolisierbare Kohlenstoffquelle, wobei die Kohlenstoffquelle in einer Konzentration von bis zu etwa 2% w/v, aber von nicht mehr als der zur Förderung normaler Entwicklung von Embryonen dieser Pflanzenspezies notwendigen Menge vorliegt, und
(ii) ein nicht-permeierendes Osmotikum, bei dem es sich nicht um eine zusätzliche Menge an metabolisierbarer Kohlenstoffquelle handelt, wobei die Konzentration des Osmotikums so gewählt ist, daß ein Wasserpotential von wenigstens 300 mmol/kg erzeugt wird.

24. Medium nach Anspruch 23, wobei die metabolisierbare Kohlenstoffquelle ausgewählt ist aus der Klasse umfassend Fructose, Glucose, Maltose, Myoinositol und Sucrose.

25. Medium nach Anspruch 23, wobei die Konzentration des Osmotikums wenigstens etwa 7% w/v beträgt.

26. Verfahren nach Anspruch 1, wobei die Embryonen Embryonen von Gymnospermen umfassen.

27. Verfahren nach Anspruch 26, wobei die Embryonen von einer Kiefernspezies sind.

## Revendications

1. Procédé pour cultiver des embryons d'une espèce de plante jusqu'à maturité en présence d'une source de carbone métabolisable, comprenant la sélection d'une quantité de ladite source de carbone métabolisable dans la plage allant d'environ la quantité nécessaire pour satisfaire les besoins nutritionnels minimaux des embryons mais n'allant pas au-delà d'environ la quantité nécessaire pour favoriser le développement normal des embryons de ladite espèce de plante, et comprenant en outre le fait de soumettre des embryons à une contrainte hydrique autres que des quantités additionnelles de sources de carbone métabolisées, pour créer un potentiel hydrique d'au moins 300 mmole / kg.

2. Procédé selon la revendication 1, dans lequel la source de carbone métabolisable est présente en une concentration inférieure à environ 2 % p / v.

3. Procédé selon la revendication 1, dans lequel la source de carbone métabolisable est présente en une concentration inférieure à environ 1 % p / v.

4. Procédé selon la revendication 1, dans lequel les embryons sont sélectionnés dans la classe comprenant les embryons d'angiosperme.

5. Procédé selon la revendication 4, dans lequel les embryons sont sélectionnés dans la classe comprenant les embryons d'angiosperme dicotylédone.

6. Procédé selon la revendication 5, dans lequel les embryons appartiennent au genre *Brassica*.

7. Procédé selon la revendication 6, dans lequel les embryons appartiennent à l'espèce *napus*.

8. Procédé selon la revendication 1, dans lequel les embryons sont sélectionnés dans la classe comprenant les embryons somatiques, zygotiques et dérivés de microspores.

9. Procédé selon la revendication 8, dans lequel les embryons sont dérivés de microspores.

10. Procédé selon la revendication 1, dans lequel ledit potentiel hydrique est au moins environ 750 mmole / kg.

11. Procédé selon la revendication 1, dans lequel le moyen de contrainte hydrique est sélectionné parmi la classe comprenant l'effet environnemental, les agents de contrainte hydrique et les combinaisons de ceux-ci.

12. Procédé selon la revendication 11, dans lequel l'agent de contrainte hydrique comprend au moins un gel.

13. Procédé selon la revendication 11, dans lequel l'agent de contrainte hydrique comprend au moins un osmoticum.

14. Procédé selon la revendication 13, dans lequel l'osmoticum est sélectionné parmi le groupe comprenant les osmoticum non perméables.

15. Procédé selon la revendication 13, dans lequel l'osmoticum a une taille moléculaire d'au moins environ 30 unités Angstrom (Å).

16. Procédé selon la revendication 13, dans lequel l'osmoticum est sélectionné parmi le groupe comprenant les polyalkylènes glycoles.

17. Procédé selon la revendication 16, dans lequel l'osmoticum comprend au moins un polyalkylène glycole ayant un poids moléculaire minimum d'environ 1 000.

18. Procédé selon la revendication 11, dans lequel la contrainte hydrique environnementale est appliquée au moins en partie par une humidité relative située dans la plage de 5 % à 100 %.

19. Procédé selon la revendication 1, dans lequel ladite source de carbone métabolisable est sélectionnée parmi le groupe comprenant le fructose, le glucose, le maltose, le méso-inositol et le saccharose.

20. Procédé selon la revendication 1, dans lequel ladite source de carbone métabolisable est sélectionnée parmi le groupe comprenant des sources de carbone faiblement métabolisées.

21. Procédé selon la revendication 20, dans lequel ladite source de carbone faiblement métabolisée est un alcool de sucre.

22. Procédé selon la revendication 20, dans lequel la concentration de ladite source de carbone est suffisamment faible pour minimiser les effets toxiques sur les embryons.

23. Milieu pour cultiver des embryons de plantes d'une espèce de plante jusqu'à maturité, comprenant :
(i) une source de carbone métabolisable, ladite source de carbone étant présente en une concentration allant jusqu'à environ 2 % p / v, mais n'étant pas supérieure à la quantité nécessaire pour favoriser le développement normal des embryons de ladite espèce de plante, et
(ii) un osmoticum non perméable, autre qu'une quantité supplémentaire de source de carbone métabolisable, la concentration dudit osmoticum étant sélectionnée pour créer un potentiel hydrique d'au moins 300 mmole / kg.

24. Procédé selon la revendication 1, dans lequel la source de carbone métabolisable est sélectionnée parmi la catégorie comprenant le fructose, le glucose, le maltose, le méso-inositol et le saccharose.

25. Milieu selon la revendication 1, dans lequel la concentration dudit osmoticum est au moins d'environ 7 % p / v.

26. Procédé selon la revendication 1, dans lequel lesdits embryons comprennent des embryons de gymnosperme.

27. Procédé selon la revendication 26, dans lequel lesdits embryons sont des embryons d'une espèce de pin.
